# DEMANDE DE BREVET EUROPEEN

(11) **EP 2 243 787 A2**
(43) Date de publication de la demande: **27.10.2010**
(21) Numéro de dépôt: 10155242.0
(22) Date de dépôt: 13.10.2003
(51) Int. Cl.: C07K 14/075, C07K 14/47, C07K 16/08, C07K 16/18, C12N 15/12, C12N 15/34, C12N 15/63, A61K 38/16, A61K 38/17, A61P 35/00, G01N 33/68

(54) **Peptides liant la protéine phosphatase 2a et polynucléotides les codant**

(30) Priorité: 16.10.2002 CA 2408207
(62) Demande divisionnaire de: 03780221.2
(71) Demandeur: Institut Pasteur, 75724 Paris Cédex 15 (FR)
(72) Inventeur: Garcia, Alphonse, 92120 Montrouge (FR); Dessauge, Frédéric, 35160 Montfort sur Meu (FR); Cayla, Xavier, 37210 Rochecorbon (FR); Rebollo, Angelita, 75015 Paris (FR)
(74) Mandataire: Desaix, Anne

(57) **Abrégé**

L'invention a trait à de nouveaux peptides E4orf4 ou Bcl-2 synthétiques ou naturels utiles en particulier dans les traitements anti-tumoraux, antiviraux et antiparasitaires, lesdits peptides étant de tailles inférieure à 33 acides aminés et **caractérisés en ce qu**'ils lient *in vitro* une holoenzyme protéine phosphatase 2A ou l'une de ses sous-unités. L'invention a aussi trait aux polynucléotides encodant les nouveaux peptides, aux vecteurs les exprimant, ainsi qu'aux anticorps les reconnaissant et aux sondes reconnaissant leurs transcrits.

SEQUENCE LISTING

<110>  INSTITUT PASTEUR

<120>  PEPTIDES LIANT LA PROTEINE PHOSPHATASE 2A ET POLYNUCLEOTIDES LES
       CODANT

<130>  B05776AAC AD/SL/CAL

<140>  EP XXXX
<141>  2010-03-02

<150>  CA 2.408 207
<151>  2002-10-16

<160>  15

<170>  PatentIn version 3.3

<210>  1
<211>  24
<212>  PRT
<213>  Artificial

<220>
<223>  Peptide

<400>  1

Arg Glu Leu Gly Ser Met Leu Glu Ser Pro Met Glu Phe Leu Phe Asp
1               5                   10                  15

Thr Ile Asp Asp Val Thr Ala Ser
            20

<210>  2
<211>  22
<212>  PRT
<213>  Artificial

<220>
<223>  Peptide

<400>  2

Leu Gly Ser Met Leu Glu Ser Pro Met Glu Phe Leu Phe Asp Thr Ile
1               5                   10                  15

Asp Asp Val Thr Ala Ser
            20

<210>  3
<211>  20
<212>  PRT
<213>  Artificial

<220>
<223>  Peptide

<400>  3

Ser Met Leu Glu Ser Pro Met Glu Phe Leu Phe Asp Thr Ile Asp Asp
1               5                   10                  15

Val Thr Ala Ser
            20


<210>  4
<211>  16
<212>  PRT
<213>  Artificial

<220>
<223>  Peptide

<400>  4

His Phe Lys Gly Thr Leu Ile Lys Glu Ile Ser Asp Val Val Asn Asn
1               5                   10                  15


<210>  5
<211>  18
<212>  PRT
<213>  Artificial

<220>
<223>  Peptide

<400>  5

Ala Phe Arg Gly Arg Glu Val Thr Val Ser Leu Leu Glu Val Phe Ser
1               5                   10                  15


Phe Cys



<210>  6
<211>  32
<212>  PRT
<213>  Artificial

<220>
<223>  Peptide

<400>  6

Val Lys Lys Lys Lys Ile Lys Arg Glu Ile Lys Ile Ser Met Leu Glu
1               5                   10                  15


Ser Pro Met Glu Phe Leu Phe Asp Thr Ile Asp Asp Val Thr Ala Ser
            20                  25                  30


<210>  7
<211>  20
<212>  PRT
<213>  Artificial

<220>
<223>  Peptide

<400>  7

Ala Arg Thr Ser Pro Leu Gln Thr Pro Ala Ala Pro Gly Ala Ala Ala
1               5                   10                  15


Gly Pro Ala Leu

            20

<210>  8
<211>  16
<212>  PRT
<213>  Artificial

<220>
<223>  Peptide

<400>  8

Arg Phe Ala Thr Val Val Glu Glu Leu Phe Arg Asp Gly Val Asn Trp
1               5                   10                  15


<210>  9
<211>  22
<212>  PRT
<213>  Artificial

<220>
<223>  Peptide

<400>  9

Arg Pro Leu Phe Asp Phe Ser Trp Leu Ser Leu Lys Thr Leu Leu Ser
1               5                   10                  15


Leu Ala Leu Val Gly Ala
            20


<210>  10
<211>  16
<212>  PRT
<213>  Artificial

<220>
<223>  Peptide

<400>  10

Pro Leu Gln Thr Pro Ala Ala Pro Gly Ala Ala Ala Gly Pro Ala Leu
1               5                   10                  15


<210>  11
<211>  20
<212>  PRT
<213>  Artificial

<220>
<223>  Peptide

<400>  11

Leu Phe Asp Phe Ser Trp Leu Ser Leu Lys Thr Leu Leu Ser Leu Ala
1               5                   10                  15


Leu Val Gly Ala
            20


<210>  12
<211>  375

<212>  DNA
<213>  Artificial

<220>
<223>  Polynucleotide E4 orf4

<400>  12
atggcccacc atcgtctgcc ccgcgtttgt gtaaagggca ttattcattt tgaggaagat      60

tttgttagag agcttggatc aatgctggag tctcctatgg agtttctctt cgacaccatt     120

gatgatgtta ctgcttctat attttgtgaa agcatgttta aggctgttga taaaaacaag     180

cctgggatta cttttaaggt ggtcttttac tctcagcttg gttttgagta tgatgatgct     240

cttgcacatt ttaaaggcac tttaattaaa gaaatttctg acgttgttaa taatcaccct     300

aatgtaaaca atgcttttag aggaagggag attgtcactg tatctttgtt agaagtgttt     360

agtttttgtt cataa                                                      375

<210>  13
<211>  618
<212>  DNA
<213>  Artificial

<220>
<223>  Polynucleotide Bcl-2

<400>  13
atggcgcacg ctgggagaac ggggtacgac aaccgggaga tagtgatgaa gtacatccat      60

tataagctgt cgcagagggg ctacgagtgg gatgcgggag atgtgggcgc cgcgcccccg     120

ggggccgccc ccgcaccggg catcttctcc tcccagcccg ggcacacgcc ccatccagcc     180

gcatcccgcg acccggtcgc caggacctcg ccgctgcaga ccccggctgc ccccggcgcc     240

gccgcggggc ctgcgctcag cccggtgcca cctgtggtcc acctggccct ccgccaagcc     300

ggcgacgact tctcccgccg ctaccgcggc gacttcgccg agatgtccag ccagctgcac     360

ctgacgccct tcaccgcgcg gggacgcttt gccacggtgg tggaggagct cttcagggac     420

ggggtgaact gggggaggat tgtggccttc tttgagttcg gtggggtcat gtgtgtggag     480

agcgtcaacc gggagatgtc gcccctggtg gacaacatcg ccctgtggat gactgagtac     540

ctgaaccggc acctgcacac ctggatccag gataacggag gctgggtagg tgcatctggt     600

gatgtgagtc tgggctga                                                   618

<210>  14
<211>  128
<212>  PRT
<213>  Artificial

<220>
<223>  E4orf4 polypeptide

<400>  14

Met Ala His His Arg Leu Pro Arg Val Cys Val Lys Gly Ile Ile His
1               5                   10                  15

Phe Glu Glu Asp Phe Val Arg Glu Leu Gly Ser Met Leu Glu Ser Pro

20                  25                  30

Met Glu Phe Leu Phe Asp Thr Ile Asp Asp Val Thr Ala Phe Thr Ser
        35                  40                  45

Ile Phe Cys Glu Ser Met Phe Lys Ala Val Asp Lys Asn Lys Pro Gly
    50                  55                  60

Ile Thr Phe Lys Val Val Phe Tyr Ser Gln Leu Gly Phe Glu Tyr Asp
65                  70                  75                  80

Asp Ala Leu Ala His Phe Lys Gly Thr Leu Ile Lys Glu Ile Ser Asp
                85                  90                  95

Val Val Asn Asn His Pro Asn Val Asn Asn Phe Thr Ala Phe Arg Gly
            100                 105                 110

Arg Glu Ile Val Thr Val Ser Leu Leu Glu Val Phe Ser Phe Cys Ser
        115                 120                 125

<210>  15
<211>  211
<212>  PRT
<213>  Artificial

<220>
<223>  Bcl-2 polypeptide

<400>  15

Met Ala His Ala Gly Arg Thr Gly Tyr Asp Asn Arg Glu Ile Val Met
1               5                   10                  15

Lys Tyr Ile His Tyr Lys Leu Ser Gln Arg Gly Tyr Glu Trp Asp Ala
            20                  25                  30

Gly Asp Val Gly Ala Ala Pro Pro Gly Ala Ala Pro Ala Phe Thr Pro
        35                  40                  45

Gly Ile Phe Ser Ser Gln Pro Gly His Thr Pro His Pro Ala Ala Ser
    50                  55                  60

Arg Asp Pro Val Ala Arg Thr Ser Pro Leu Gln Thr Pro Ala Ala Pro
65                  70                  75                  80

Gly Ala Ala Ala Gly Pro Ala Leu Ser Pro Val Pro Pro Val Val His
                85                  90                  95

Leu Ala Leu Arg Gln Ala Gly Asp Asp Phe Phe Thr Ser Arg Arg Tyr
            100                 105                 110

Arg Gly Asp Phe Ala Glu Met Ser Ser Gln Leu His Leu Thr Pro Phe
        115                 120                 125

Thr Ala Arg Gly Arg Phe Ala Thr Val Val Glu Glu Leu Phe Arg Asp
    130                 135                 140

Gly Val Asn Trp Gly Arg Ile Val Ala Phe Phe Glu Phe Gly Gly Val
145                 150                 155                 160

Met Cys Val Glu Ser Val Asn Phe Thr Arg Glu Met Ser Pro Leu Val
                165                 170                 175

Asp Asn Ile Ala Leu Trp Met Thr Glu Tyr Leu Asn Arg His Leu His
            180                 185                 190

Thr Trp Ile Gln Asp Asn Gly Gly Trp Val Gly Ala Ser Gly Asp Val
        195                 200                 205

Ser Leu Gly
    210

## Description

### DOMAINE DE L'INVENTION

La présente invention concerne des peptides liant la protéine phosphatase 2A, une cible importante pour le contrôle de l'apoptose, notamment dans les cellules cancéreuses, ainsi que pour le contrôle des infections virales et parasitaires.

### DESCRIPTION DE L'ART ANTÉRIEUR

Étant donné le rôle des peptides de l'invention dans la modulation de l'activité de la protéine phosphatase 2A cellulaire, il est important de rappeler l'état des connaissances actuelles sur les protéines phosphatases.2A, leur rôle physiologique et leurs interactions avec certaines protéines cellulaires, virales ou parasitaires.

La physiologie de la cellule est contrôlée en partie par la modulation de l'état de phosphorylation des protéines. L'état de phosphorylation des protéines cellulaires dépend de l'action antagoniste des protéines kinases qui les phosphorylent et des protéines phosphatases qui les déphosphorylent.

Les protéines phosphatases sont divisées en deux groupes principaux: les tyrosine phosphatases et les sérine/thréonine phosphatases. Les sérine/thréonine phosphatases sont classées en deux catégories selon la spécificité de leur substrat et leur sensibilité à certains inhibiteurs, les phosphatases de type 1 (PP1) et les phosphatases de type 2 (PP2). Les phosphatases de type 2 se divisent encore en différentes classes, incluant la phosphatase 2A (PP2A), la phosphatase 2B (PP2B) ou la calcineurine dont l'activité est régulée par le calcium, et la phosphatase 2C (PP2C) dont l'activité est dépendante du magnésium.

*In vivo* les serine/thréonine protéines phosphatases PP1 et PP2A forment deux familles de plusieurs holoenzymes d'expression ubiquitaire qui sont produites par l'interaction spécifique entre leurs sous-unités catalytiques (PP1 c et PP2Ac) et une grande variété de sous-unités régulatrices, et qui sont impliquées dans le ciblage et/ou la régulation de l'activité phosphatasique (pour une revue récente, voir Garcia A. et al., PP1 et PP2A des ser/thr phosphatases au coeur de l'apoptose (2001) Med/Sci 17, 1214-1216).

On sait maintenant que les phosphatases de type 2A sont très conservées au cours de l'évolution et sont potentiellement activées dans la régulation de nombreux processus biologiques. Les enzymes PP2A ont clairement été impliquées dans la régulation de la transcription, le contrôle du cycle cellulaire, et la transformation virale. En outre, les PP2A sont la cible de différentes protéines virales ou parasitaires, suggérant un rôle des PP2A dans les interactions hôtes-pathogènes.

Les PP2A sont des complexes oligomériques (holoenzymes) comprenant chacun, une sous-unité catalytique C et une ou deux sous-unités régulatrices, (A) et (B). La structure de la sous-unité (A) consiste en 15 répétitions imparfaites d'une séquence d'acides aminés conservée de 38 à 40 acides aminés, dont certaines interagissent avec les sous-unités (B) et (C). Les sous-unités (A) et (C), conservées au cours de l'évolution, forment la structure de base de l'enzyme et sont exprimées constitutivement. Au contraire, les sous-unités (B) constituent une famille de protéines régulatrices sans structure commune et différentiellement exprimées (Cohen P. The structure and regulation of protein phosphatases. Annu. Rev. Biochem. 1989; 58:453-508). Ainsi, les protéines phosphatases 2A existent *in vivo* sous deux formes différentes: une forme dimérique (AC) et une forme trimérique (ABC). Les sous-unités (B) régulent l'activité phosphatasique et la spécificité vis-à-vis du substrat. L'existence de formes multiples de PP2A est corrélée à des fonctions distinctes et variées des PP2A *in vivo.*

Récemment, différentes protéines non cellulaires, et en particulier des protéines virales et parasitaires, ont été impliquées dans la modulation de certaines activités spécifiques des protéines phosphatases 2A.

Différentes stratégies impliquant la PP2A ont été adoptées par les virus afin de faciliter leur réplication et leur survie dans la cellule de l'hôte. Par exemple, le *parainfluenza* virus incorpore dans sa particule virale la protéine PKC ζ, protéine d'origine cellulaire sous le contrôle de la PP2A. Ceci lui permet de perturber la phosphorylation des protéines de son hôte et de faciliter sa propre réplication (B.P. Gupta et al. Cellular protein kinase C ζ regulates human parainfluenza virus type 3 replication. Proc. Natl. Acad. Sci. USA 1995; 92:5204-8).

Plusieurs virus à ADN ayant un pouvoir transformant, tels que les *papovaviridae* ou les adénovirus, de même que certains rétrovirus, tels que le virus de l'imuno déficience humaine de type 1 (VIH-1) codent pour des protéines qui interagissent directement avec certaines PP2A de l'hôte. Tous ces virus comprennent des protéines qui bien que structurellement différentes, interagissent avec certaines holoenzymes et en modifient l'activité phosphatasique.

Il a été montré en particulier que la protéine E4orf4 des adénovirus se lie à une PP2A hétérotrimérique, et plus précisément, à une sous-unité régulatrice (B), ce qui entraîne une diminution de la transcription de JunB dans la cellule infectée. Cet effet pourrait jouer un rôle important durant l'infection virale en régulant la réponse apoptotique des cellules infectées. De manière intéressante, il a aussi été montré que l'interaction de E4orf4 avec la PP2A induit l'apoptose de cellules transformées de manière p53-indépendante (Shtrichman R. et al. Adenovirus type 5 E4 open reading frame 4 protein induces apoptosis in transformed cells. J. Virol. 1998; 72 : 2975-82).

Les virus oncogènes à ADN de la famille des *Papovaridae*, incluant SV40 et le virus du polyome, induisent la transformation cellulaire. Il a été montré que la PP2A interagit avec les antigènes « petit T » de SV40 et « petit T » et « T moyen » du virus polyome. Ces interactions de protéines virales avec la PP2A ont été clairement impliquées dans la transformation virale. Enfin, la régulation transcriptionnelle, un processus normalement réalisé dans la cellule par les différents facteurs se fixant spécifiquement sur des séquences régulatrices promotrices, représente probablement le mécanisme le plus important impliqué dans le contrôle de l'expression virale par la PP2A. Ainsi, il a été démontré que la PP2A est un régulateur négatif de nombreux facteurs de transcription impliqués notamment dans les processus de croissance et de prolifération cellulaire, incluantAP1/SRE, NF-kB, Sp1 et CREB (Waszinski, B.E. et al. Nuclear protein phosphatase 2A dephosphorylates protein kinase A-phosphorylated CREB and regulates CREB Transcriptional stimulation. Mol. Cell Biol. 1993 :13, 2822-34). La régulation virale de ces facteurs de transcription permettrait de moduler la transcription virale.

La protéine virale de VIH-1, *Vpr*, interagit *in vitro* avec la PP2A et en stimule l'activité catalytique (Tung L, et al., Direct activation of protein phosphatase 2A0 by HIV-1 encoded protein complex Ncp7 :vpr. FEBS Lett 1997; 401: 1997-201). *Vpr* peut induire l'arrêt en G2 de cellules infectées en inhibant l'activation du complexe p34cdc2-cycline B. D'autre part, *Vpr* interagit avec le facteur de transcription Sp1 et est un trans-activateur faible de la transcription de VIH-1 Sp1 dépendante. Ainsi, la protéine *Vprde* VIH-1, qui est incorporée dans le virion, serait impliquée *in vivo* dans l'initiation de la transcription virale, une étape évidemment essentielle pour réguler l'expression du facteur de transcription Tat (un régulateur majeur de la transcription codé par le virus VIH-1).

Au contraire du rôle bien établi des protéines kinases dans les infections parasitaires, c'est seulement au cours des dernières années que les sérine/thréonine phosphatases ont commencé à être reconnues comme étant des régulateurs potentiels importants dans le domaine de la parasitologie.

L'absence de motifs communs à l'ensemble des protéines interagissant avec PP2A empêche la simple identification bio-informatique des motifs peptidiques directement impliqués dans la liaison de ces protéines avec PP2A.

Or, étant donné le rôle majeur des protéines phosphatases 2A dans les interactions virus-hôtes ou parasites-hôtes tel que résumé ci-dessus, on comprend l'intérêt d'identifier les sites de liaison des protéines virales ou parasitaires avec les holoenzymes PP2A ou l'une de leurs sous-unités, afin d'identifier de nouvelles cibles thérapeutiques pour ces pathogènes, viraux ou parasitaires.

Les sérine/thréonine protéine phosphatases de type 1 (PP1) et de type 2A (PP2A) représentent des nouvelles cibles potentiellement importantes pour le contrôle de l'apoptose, notamment dans les cellules cancéreuses, ainsi que pour le contrôle des infections virales ou parasitaires (pour revue voir A. Garcia et al. (2000). Protein Phosphatase 2A: a definite player in Viral and parasitic regulation. Microbes Inf. 2,401-407 ; Et X. Cayla et al. (2000). La Protéine Phosphatase 2A: une nouvelle piste pour l'étude des virus et des parasites. Méd/Sci 16, 122-127). Plus particulièrement, un rôle crucial de PP1/PP2A serait au niveau de la régulation des protéines anti-apoptotiques Bcl-2 et de la survie cellulaire (Garcia A. et al., PP1 et PP2A des ser/thr phosphatases au coeur de l'apoptose (2001). Med/Sci 17, 1214-1216; Ayllôn, V. et al. (2000). Protein phophatase 1- is a Ras-activated Bad phosphatase that regulates IL-2 deprivation-induced apoptosis. EMBO J. 19, 2237-2246, Ayllôn, V, et al.(2001) Bcl-2 targets protein phosphatase 1 alpha to Bad. J. Immunol. 15; 166:7345-7352).

De manière générale, l'identification des peptides interagissant avec PP2A permettrait de produire de nouveaux médicaments susceptibles de bloquer par inhibition compétitive les mécanismes cellulaires induits par les protéines virales ou parasitaires via leur interaction avec PP2A et en particulier les mécanismes d'infection, de prolifération des pathogènes et de transformation néoplasique des cellules.

En particulier, il a été rapporté que l'activation de PP2A après interaction avec la protéine adenovirale E4orf4, induit l'apoptose dans les cellules transformées (Shtrichman R, et al. (2000) Oncogene. 19, 3757-3765). Cet effet qui est spécifique requiert une interaction avec la sous-unité B alpha (Bα) de PP2A (Marcellus et al. J Virol. (2000) 74:7869-7877)/ Goedert et al. J.Neurochem (2000) 75,2155-2162)). Au total l'ensemble de ces observations suggère l'hypothèse que l'interaction de peptides mimant le site ABC1 et/ou A3 avec PP2A pourrait induire l'apoptose des cellules transformées.

WO901563 ET WO0104629 A1 décrivent la protéine E4orf4 humaine et son rôle dans l'induction de l'apoptose des cellules tumorales, particulièrement lorsqu'elle est exprimée à l'aide d'un vecteur adénoviral. WO0104629 A1 concerne d'ailleurs des polypeptides modulateurs et mimétiques E4orf4 et PP2A permettant d'induire une mort cellulaire sélective. Ce document divulgue une invention qui concerne l'aptitude de la protéine adénovirale E4orf4 à provoquer la mort de cellules néoplasiques, mais pas celle de cellules non néoplasiques. D'autre part, WO9801563 A2 concerne les protéines E4orf4 et E4orf6 d'adénovirus destinées à induire la mort cellulaire. Enfin, la demande de brevet FR 0110139 décrit des composés peptidiques présentant la propriété de liaison avec la PP2A.

Également, les protéines de la famille Bcl-2 qui, chez les mammifères, comprend une vingtaine de membres, peuvent être divisées en trois sous-familles comprenant:
- les membres anti-apoptotiques (du type Bcl-2 lui même) qui possèdent tous au moins quatre motifs conservés, appelés "BH1 à BH4" pour "Bcl-2 Homology domain", sont nécessaires à la fonction de survie cellulaire. Le motif BH4 contient le domaine d'interaction avec les protéines Raf et Apaf-1, et avec la calcineurine;
- les membres pro-apoptotiques de type Bax ne contiennent pas de domaine BH4; et
- les membres pro-apoptotiques de type Bad ne possèdent qu'un domaine BH3.

Des expériences de mutagenèse montrent que l'activité anti-apoptotique de Bcl-2 requiert sa phosphorylation au niveau d'un résidu particulier, la sérine 70, dont le remplacement par l'alanine inhibe la survie. Par ailleurs les travaux de l'équipe du Dr May (USA) (2001, Vol. 15, No. 4, pp 515-522) suggèrent qu'en présence d'IL-3, PP2A pourrait s'associer de façon transitoire à Bcl-2. L'utilisation d'un mutant ponctuel (où un résidu alanine remplace la sérine 70) indique que la liaison de PP2A à Bcl-2 requiert la sérine 70 qui par conséquent pourrait appartenir au site de liaison. Les auteurs suggèrent donc un mécanisme de régulation dynamique où PP2A serait une Bcl-2 phosphatase antagoniste des Bcl-2 kinases, par exemple les PKC (pour discussion, voir Garcia A. et al.PP1 et PP2A des ser/thr phosphatases au coeur de l'apoptose (2001). Med/Sci 17, 1214-1216.

La présence de ces peptides Bcl-2 à l'intérieur de la cellule pourrait donc déréguler la phosphorylation et donc l'activité de Bcl-2 ce qui en conséquence pourrait contrecarrer le développement des tumeurs Bcl-2 dépendantes.

En bref, il existe donc un besoin au niveau des traitements anti-tumoraux, antiviraux et antiparasitaires pour des peptides dérivés des séquences E4orf4 et Bcl-2 liant la PP2A ou l'une de ses sous-unités.

### SOMMAIRE DE L'INVENTION

L'invention s'intéresse à des peptides E4orf4 et Bcl-2 de taille réduite, liant une holoenzyme PP2A ou l'une de ses sous-unités. Au contraire des protéines E4orf4 et Bcl-2 natives ou de domaines polypeptidiques de taille importante, des peptides de taille réduite ont l'avantage d'être aisément synthétisés, par voie chimique ou en systèmes cellulaires, avec un rendement important et un coût réduit. Les peptides de l'invention présentent, en outre, l'avantage d'être plus accessibles et plus facilement transférés dans le cytoplasme ou dans le noyau des cellules à l'aide de vecteurs appropriés, en vue d'une utilisation thérapeutique.

L'invention découle de l'identification de peptides E4orf4 et Bcl-2, d'une taille inférieure à 30 acides aminés, et notamment des peptides d'une taille inférieure à 20 acides aminés, interagissant *in vitro* avec une holoenzyme PP2A purifiée ou l'une de ses sous-unités.

En particulier, les inventeurs ont identifié par la technique des "SPOT synthesis" décrite par Frank et Overwing (Methods in Molecular Biology, 1996, vol. 66: 149-169, Epitope Mapping Protocols , G.E. Morris Humana Press Inc., Totowa New Jersey) les sites de liaison des protéines E4orf4 (d'adénovirus de type 2 de chien) et Bcl-2 interagissant avec une holoenzyme PP2A ou l'une de ses sous-unités.

D'une part, les peptides de l'invention sont des peptides d'une taille inférieure à 30 acides aminés, interagissant *in vitro* avec une holoenzyme PP2A purifiée ou l'une de ses sous-unités, lesdits peptides étant dérivés de la protéine E4orf4 (d'adénovirus de type 2 de chien) et Bcl-2. Des antagonistes dérivés de ces peptides et sélectionnés parce qu'ils inhibent l'interaction des protéines virales ou parasitaires avec une holoenzyme particulière de PP2A pourraient ainsi constituer de nouveaux agents anti-tumoraux, antiviraux ou antiparasitaires.

D'autre part, l'invention concerne également un peptide comprenant d'une part, une séquence de 12 acides aminés dérivée de Ck2α de *Theileria parva* (FD6) capable d'interagir avec la sous-unité A de la PP2A et de pénétrer dans la cellule de la lignée *Hela* (García,A. *et al*. (2000) et, d'autre part, la séquence correspondant au site d'interaction de la PP2A avec la sous-unité Bα de la PP2A site 1-B (cf. tableau 1). Ce peptide pourrait pénétrer dans les cellules et, à l'instar de la protéine E4orf4 de l'adénovirus humain, pourrait interagir avec la PP2A et provoquer l'apoptose dans les cellules cancéreuses sans affecter les cellules normales.

La méthode d'identification, décrite dans la demande FR no. 0110139 déposée le 27 juillet 2001 au nom du même demandeur, comprend les étapes suivantes consistant à:
a) déposer sous forme de spots, sur un support, des peptides E4orf4 ou Bcl-2 dont la séquence est issue respectivement de la protéine virale E4orf4 ou cellulaire Bcl-2, chaque spot correspondant au dépôt d'un peptide de séquence définie,
b) mettre en contact le support solide avec une solution contenant une holoenzyme protéine phosphatase 2A ou l'une de ses sous-unités dans des conditions permettant aux peptides présents sur le support, de lier l'holoenzyme ou l'une de ses sous-unités, et,
c) à identifier sur le support solide le peptide E4orf4 ou Bcl-2 sur lequel se fixe la protéine phosphatase 2A ou l'une de ses sous-unités.

Selon l'étape a), différents peptides sont déposés sur un support solide à des positions définies ("spot"), chaque position correspondant à une séquence peptidique spécifique et l'ensemble formant ainsi un réseau de peptides ("array") à deux dimensions.

Différentes méthodes de préparation de tels réseaux ont été décrites récemment (pour revue, voir Figeys et Pinto, 2001 Electrophoresis 22: 208-216; et Walter et al., 2000 Curr Opin Microbiol 3 : 298-302). L'ensemble de ces méthodes comprend en général la fixation covalente de peptides sur un support, en particulier à l'aide de lieurs (linkers) chimiques. À titre d'exemple, l'homme du métier pourra notamment se reporter à la technique des "SPOT synthesis" consistant à synthétiser directement sur une membrane de cellulose, des peptides comprenant jusqu'à 20 résidus (Frank et Overwing, Methods in Molecular Biology, 1996, vol. 66: 149-169, Epitope Mapping Protocols, G.E. Morris Humana Press Inc., Totowa New Jersey).

De manière générale, toute méthode peut être utilisée dès lors que celle-ci permet l'obtention d'un réseau de peptides, E4orf4 ou Bcl-2, déposés sur un support solide, utilisable pour détecter des interactions spécifiques entre les peptides déposés et l'holoenzyme PP2A ou l'une de ses sous-unités.

L'ensemble des séquences de peptides E4orf4 et Bcl-2 déposés recouvre la séquence complète de la protéine virale ou cellulaire dont ces séquences sont issues. Ainsi, le procédé permet de tester en une seule étape la séquence complète, celle-ci étant "sectionnée" en un nombre défini de peptides, de séquences généralement chevauchantes.

Les peptides déposés sous forme de spot sont d'une taille inférieure à 20 acides aminés, et mieux, d'une taille inférieure à 15 acides aminés.

Les peptides peuvent aussi être déposés sur une membrane de cellulose.

Le réseau ainsi obtenu est mis en contact à l'étape b), avec une holoenzyme protéine phosphatase de type 2A ou l'une de ses sous-unités.

Par "holoenzyme protéine phosphatase de type 2A", il faut comprendre tout complexe dimérique (AC) ou hétérotrimérique (ABC), purifié d'un extrait cellulaire ou reconstitué après purification des deux sous-unités (A) et (C) d'une protéine phosphatase de type (2A) et le cas échéant d'une sous-unité (B). Les protéines phosphatases de type (2A) sont de préférence issues de mammifères.

Les supports sont incubés par exemple dans une solution tampon comprenant les protéine phosphatase purifiées ou l'une de leurs sous-unités purifiées. Une solution tampon utilisable est le TBS (TRIS BUFFER SALINE) contenant 5% de lait écrémé en poudre et 3% de BSA.

Le peptide sur lequel se fixe l'holoenzyme protéine phosphatase de type 2A est identifié en général par le marquage direct ou indirect de la protéine phosphatase et l'identification des spots au niveau desquels s'est fixé la protéine marquée. La fixation de la PP2A ou l'une de ses sous-unités au niveau de l'un des spots de peptides peut ainsi être révélée en particulier à l'aide d'antisérums, selon les techniques classiquement utilisées pour le Western Blot ou le test ELISA en phase solide, après incubation du support contenant le réseau de peptides avec un anticorps dirigé contre les sous-unités (A) ou (B) ou (C) ou un mélange d'anticorps dirigé contre les sous-unités (A), (B) ou (C) de PP2A.

L'application de la méthode de « SPOT synthesis » décrite dans FR no. 0110139 conduit à l'identification de peptides E4orf4 et Bcl-2, utiles notamment dans les traitements anti-tumoraux, antiviraux et antiparasitaires, d'une taille inférieure à 30 acides aminés, voire inférieure à 20 acides aminés, lesdits peptides étant capables de lier *in vitro* une holoenzyme protéine phosphatase de type 2A ou l'une de ses sous-unités.

L'invention vise par conséquent un peptide E4orf4 ou Bcl-2, naturel ou synthétique, d'une taille inférieure à 30 acides aminés, de préférence inférieure à 20 acides aminés, caractérisé en ce qu'il lie *in vitro,* de manière spécifique, une holoenzyme protéine phosphatase de type 2A ou l'une de ses sous-unités, (A), (B) ou (C). Par liaison spécifique, il faut comprendre que le peptide est capable d'inhiber de manière compétitive la liaison d'une protéine d'origine virale ou parasitaire avec des PP2A.

L'invention vise aussi un peptide comprenant, d'une part, une séquence de 12 acides aminés dérivée de Ck2α de *Theileria parva* (FD6) capable d'interagir avec la sous-unité A de la PP2A et de pénétrer dans la cellule de la lignée HeLa (Garcia, A. *et al.* (2000) Inhibitions de processus tumoraux ou infectieux partransfert intracellulaire de peptides mimant des sites d'interaction avec la sérine /thréonine phosphatase PP2A et, d'autre part, la séquence correspondant au site d'interaction de PP2A avec la sous-unité Bα de la PP2A site 1-B (cf. Tableau 1). Ce peptide pourrait pénétrer dans les cellules et, à l'instar de la protéine E4orf4 de l'adénovirus humain, pourrait interagir avec PP2A et provoquer l'apoptose dans les cellules cancéreuses sans affecter les cellules normales.

Les peptides E4orf4 et Bcl-2 identifiés, et le peptide proposé FD6-E4orf4 sont particulièrement utiles dans le traitement de certaines tumeurs, de certaines infections virales ou parasitaires. L'homme du métier peut sélectionner, à l'aide de tests de compétition de liaison, de nouveaux peptides, dérivés des séquences identifiées de l'invention, les dits peptides inhibant de manière compétitive la liaison de la protéine native dont ils dérivent avec une holoenzyme PP2A ou l'une de ses sous-unités.

Ainsi, l'invention concerne également un peptide naturel ou synthétique, tel que défini plus haut, caractérisé en ce qu'il inhibe de manière compétitive, l'interaction de la protéine native dont il dérive avec une holoenzyme PP2A ou l'une de ses sous-unités.

Les peptides selon l'invention, pour être efficace *in vivo* dans le traitement de certaines tumeurs ou certaines infections virales ou parasitaires, peuvent être couplés à un vecteur capable de transférer ledit peptide dans une cellule eucaryote. La taille réduite des peptides de l'invention leur permet de traverser plus facilement la membrane cellulaire. L'utilisation de vecteurs appropriés permet en outre de cibler les peptides à certains tissus, certaines cellules, voire certains compartiments cellulaires particuliers, et notamment, le cytoplasme ou le noyau des cellules, en fonction de l'effet thérapeutique recherché.

L'invention porte naturellement sur les moyens permettant la synthèse des peptides de l'invention. En particulier, l'invention porte sur un polynucléotide **caractérisé en ce que** sa séquence consiste en la séquence codante d'un peptide selon l'invention. Des polynucléotides préférés sont les polynucléotides dont la séquence est choisie parmi l'une des séquences suivantes:

E4 orf4 (375 pb)

atggcccacc atcgtctgcc ccgcgtttgt gtaaagggca ttattcattt tgaggaagat 60
tttgttagag agcttggatc aatgctggag tctcctatgg agtttctctt cgacaccatt 120
gatgatgtta ctgcttctat attttgtgaa agcatgttta aggctgttga taaaaacaag 180
cctgggatta cttttaaggt ggtcttttac tctcagcttg gttttgagta tgatgatgct 240
cttgcacatt ttaaaggcac tttaattaaa gaaatttctg acgttgttaa taatcaccct 300
aatgtaaaca atgcttttag aggaagggag attgtcactg tatctttgtt agaagtgttt 360
agtttttgtt cataa

Bcl-2 (618pb)

atggcgcacg ctgggagaac ggggtacgac aaccgggaga tagtgatgaa gtacatccat 60
tataagctgt cgcagagggg ctacgagtgg gatgcgggag atgtgggcgc cgcgcccccg 120
ggggccgccc ccgcaccggg catcttctcc tcccagcccg ggcacacgcc ccatccagcc 180
gcatcccgcg acccggtcgc caggacctcg ccgctgcaga ccccggctgc ccccggcgcc 240
gccgcggggc ctgcgctcag cccggtgcca cctgtggtcc acctggccct ccgccaagcc 300
ggcgacgact tctcccgccg ctaccgcggc gacttcgccg agatgtccag ccagctgcac 360
ctgacgccct tcaccgcgcg gggacgcttt gccacggtgg tggaggagct cttcagggac 420
ggggtgaact gggggaggat tgtggccttc tttgagttcg gtggggtcat gtgtgtggag 480
agcgtcaacc gggagatgtc gcccctggtg gacaacatcg ccctgtggat gactgagtac 540
ctgaaccggc acctgcacac ctggatccag gataacggag gctgggtagg tgcatctggt 600
gatgtgagtc tgggctga

II peut être avantageux de synthétiser un polypeptide comprenant la répétition des motifs peptidiques identifiés de l'invention. Par conséquent, l'invention porte sur un polynucléotide caractérisé en ce qu'il consiste en un multimère du polynucléotide codant pour un peptide de l'invention. L'invention porte également sur un polypeptide caractérisé en ce qu'il est constitué de la répétition d'un peptide selon l'invention. L'invention porte également sur un vecteur d'expression cellulaire, caractérisé en ce qu'il comporte un polynucléotide tel que défini plus haut et des séquences régulatrices permettant l'expression d'un peptide selon l'invention dans une cellule hôte.

L'invention vise également la méthode de préparation d'un peptide tel que défini selon l'invention, comprenant la transformation d'un hôte cellulaire à l'aide d'un vecteur d'expression cellulaire tel que défini plus haut, suivi de la mise en culture de l'hôte cellulaire ainsi transformé, et la récupération du peptide dans le milieu de culture.

L'invention porte en outre sur un anticorps purifié polyclonal ou monoclonal caractérisé en ce qu'il est capable de lier de façon spécifique un peptide selon l'invention.

Des anticorps spécifiquement dirigés contre les peptides identifiés de l'invention sont obtenues, par exemple par immunisation d'un animal après injection d'un peptide selon l'invention, et récupération des anticorps produits. Un anticorps monoclonal peut être obtenu selon les techniques connues de l'homme du métier telle que la méthode des hybridomes décrites par Kohler et Milstein (1975).

Les anticorps obtenus, spécifiquement dirigés contre des cibles de la protéine phosphatase 2A trouvent leur application en particulier dans l'immunothérapie. Ils peuvent par exemple servir d'antagonistes de protéines virales ou parasitaires dirigés contre la protéine phosphatase 2A afin de bloquer le développement viral ou parasitaire.

De même, les polynucléotides codant les peptides de l'invention peuvent être directement transférés au noyau de cellules cibles, le cas échéant à l'aide de vecteurs appropriés, afin de permettre l'expression *in vivo* des peptides correspondants, lesdits peptides étant susceptibles de bloquer par inhibition compétitive une interaction spécifique entre la protéine phosphatase 2A et la protéine virale ou cellulaire dont ils dérivent.

Ainsi, l'invention porte sur une composition pharmaceutique comprenant un des éléments choisis parmi un polynucléotide selon l'invention ou un anticorps selon l'invention.

L'invention concerne également une composition pharmaceutique comprenant l'un des peptides de l'invention en combinaison avec un véhicule pharmaceutiquement acceptable.

L'invention vise en outre une utilisation d'un peptide de l'invention défini plus haut, dans la préparation d'un médicament utile dans le traitement d'une infection virale ou parasitaire.

Les peptides de l'invention peuvent être avantageusement choisis de manière à stimuler l'induction de l'apoptose liée à l'activation de la protéine phosphatase 2A cellulaire. Ainsi, l'invention concerne également l'utilisation d'un peptide selon l'invention, tel que défini plus haut, dans la préparation d'un médicament apte à induire l'apoptose de cellules cibles, et en particulier de cellules tumorales.

Le cancer se traduit par une expression spécifique des protéines dont l'activité est régulable par les séquences de peptides de l'invention. Les séquences codant les peptides de l'invention peuvent donc être utilisées comme sonde pour détecter, de manière spécifique, à partir d'ARN extrait d'un échantillon biologique d'un patient, le développement tumoral.

De même, un anticorps selon l'invention peut être utilisé pour reconnaître spécifiquement les séquences peptidiques contenues dans les protéines virales ou cellulaires exprimées lors de la tumorigénèse.

Ainsi, l'invention porte donc sur l'utilisation d'un polynucléotide selon l'invention ou d'un anticorps selon l'invention dans le diagnostic *in vitro* de cancers.

### DESCRIPTION DES FIGURES

Figure 1: Identification des sites de liaison de la protéine E4orf4 avec PP2A.
Figure 2: Identification des sites de liaison de la protéine Bcl-2 avec PP2A.

### DESCRIPTION DES MODES DE RÉALISATION PRÉFÉRÉS DE L'INVENTION

Dans un mode de réalisation préféré de l'invention, le peptide de l'invention est caractérisé en ce qu'il s'agit d'un fragment de la protéine adénovirale E4orf4, ou de la protéine cellulaire Bcl-2, ou du peptide proposé Cka2-E4orf4, ladite protéine liant *in vitro* une protéine phosphatase de type 2A ou l'une de ses sous-unités, ou d'une séquence se distinguant du fragment de protéine précédent par substitution ou délétion d'acides aminés, ladite séquence distincte conservant néanmoins les propriétés de liaison à une protéine phosphatase de type 2A ou l'une de ses sous-unités.

En particulier, une séquence distincte est une séquence peptidique augmentant l'affinité de liaison à la protéine phosphatase de type 2A ou l'une de ses sous-unités par rapport à la séquence dont elle dérive. Une autre séquence distincte telle que définie plus haut est une séquence peptidique homologue à une séquence peptidique identifiée initialement, c'est-à-dire une séquence dérivée d'une protéine d'une autre espèce que la séquence peptidique identifiée initialement, et dont la séquence primaire peut être alignée avec la séquence peptidique identifiée initialement à laide d'un programme d'alignement optimal classiquement utilisé, tel que le programme BESTFIT (Wisconsin Genetics Software Package, Genetics Computer Group, GCG). En particulier, une séquence A sera considérée comme homologue à une séquence B si lesdites séquences A et B présentent au moins 50% d'homologie, de préférence 75% d'homologie, après alignement des séquences à l'aide d'un programme d'alignement optimal tel que le programme BESTFIT. De manière encore préférée, deux séquences sont aussi considérées homologues si les séquences sont quasi-identiques à l'exception de quelques résidus pouvant représenter 10 à 20% de variabilité sur la séquence totale. Par ailleurs les acides aminés semblables par leur fonction chimique (tels que par exemple, Arg et Lys) sont considérés comme équivalents.

Un peptide de l'invention particulièrement préféré est un fragment de la protéine E4orf4 de l'adénovirus, en particulier un fragment de la protéine E4orf4 de l'adénovirus de type 2 de chien, ou une séquence se distinguant du fragment de la protéine précédent par substitution ou délétion d'acides aminés, ladite séquence distincte conservant néanmoins les propriétés de liaison à la protéine phosphatase de type 2A ou l'une de ses sous-unités.

De manière encore préférée, un peptide selon l'invention est caractérisé en ce qu'il est inclus dans l'une des séquences suivantes:
a) RELGSMLE SPMEFLFDTI DDVTAS (SEQ. ID NO:1)
b) LGSMLE SPMEFLFDTI DDVTAS (SEQ. ID NO:2)
c) SMLE SPMEFLFDTI DDVTAS (SEQ. ID NO:3)
d) HFKGTLIKEISDVVNN (SEQ. ID NO:4)
e) AFRGRE VTVSLLEVFSFC (SEQ. ID NO: 5)
f) V K K K K I K R E I K I SMLE SPMEFLFDTI DDVTAS (SEQ. ID NO:6)
g) ARTSPLQTPAAPGAAAGPAL (SEQ. ID NO:7)
h) RFATVVEELFRDGVNW (SEQ. ID NO:8)
i) RPLFDFSWLSLKTLLSLALVGA (SEQ. ID NO:9)
j) PLQTPAAPGAAAGPAL (SEQ. ID NO:10)
k) LFDFSWLSLKTLLSLALVGA (SEQ. ID NO:11)
l) une séquence se distinguant de SEQ ID NO:1, 2, 3, 4, 5, 6, 7, 8, 9, 10 ou 11 par substitution ou délétion d'acides aminés, ladite séquence distincte conservant néanmoins les propriétés de liaison pour la protéine phosphatase de type 2A ou l'une de ses sous-unités.

Parmi les peptides de séquences se distinguant de SEQ ID NO:1, 2, 3, 4, 5 ou 6 par substitution ou délétion d'acides aminés, et entrant dans le cadre de l'invention, on citera plus particulièrement les peptides dont la séquence est incluse dans l'une des séquences de la protéine E4orf4 des différents variants de l'adénovirus de type 2 de chien et d'humain, et correspondant aux séquences homologues chez ces variants de SEQ ID NO: 1, 2, 3,4, 5 ou 6.

Parmi les peptides de séquences se distinguant de SEQ ID NO: 7, 8, 9, 10 ou 11 par substitution ou délétion d'acides aminés, et entrant dans le cadre de l'invention, on citera plus particulièrement les peptides dont la séquence est incluse dans l'une des séquences de la protéine cellulaire Bcl-2, et correspondant aux séquences homologues chez des variants de SEQ ID NO: 7, 8, 9, 10 ou 11.

Un peptide préféré selon l'invention est un peptide choisi parmi l'une des séquences SEQ ID NO:1, 2, 3, 4, 5, 6, 7, 8, 9, 10 ou 11 et **caractérisé en ce que** son administration induit l'apoptose des cellules et en particulier des cellules tumorales.

L'invention vise préférentiellement l'utilisation d'un peptide dont la séquence dérive d'un fragment de la protéine E4orf4, tel que défini plus haut, dans la préparation d'un médicament apte à inhiber l'infection virale.

### EXEMPLES

### Les protéines PP2A purifiées

La protéine trimérique PP2A1 a été purifiée à homogénéité à partir de cerveau de porc.

La protéine A (sous -unité structurale de PP2A) recombinante exprimée dans la bactérie.

La protéine B55 (sous -unité régulatrice de PP2A) recombinante exprimée dans la bactérie.

### Identification des sites de liaison de E4orf4 et de Bcl-2 avec PP2A

Nous avons cartographié le site de liaison entre les protéines E4orf4 (codée par l'adenovirus de type 2 de chien) et la protéine anti-apoptotique Bcl-2 avec PP2A en utilisant la technique des « peptides spot » qui a été précédemment décrite (Frank et Overwing. (1996). Meth. Mol. Biol. 66,149-169).

Cette approche est basée sur l'utilisation de membranes où sont déposés des dodécapeptides qui représentent la totalité de la protéine d'intérêt (ici E4orf4 et Bcl-2) avec un décalage de deux acides aminés par peptide.

Chaque membrane est d'abord saturée 1 heure à température ambiante avec du TBS contenant 5% de lait écrémé en poudre et 3% de BSA, puis incubée une nuit dans le même tampon en présence de 4µg/ml de protéine purifée (sous-unité A de PP2A et holoenzyme PP2A1). Cinquante six dodécapeptides recouvrant toute la séquence de la protéine E4orf4 et cent quinze dodécapeptides recouvrant toute la séquence de la protéine Bcl-2 ont été synthétisés et liés de façon covalente à des membranes de cellulose.

E4orf4 :
MAHHRLPRVCVKGIIHFEEDFVRELGSMLESPMEFLFDTIDDVTAFTSIFCESMFK
AVDKNKPGITFKVVFYSQLGFEYDDALAHFKGTLIKEISDVVNNHPNVNNFTAFR
GREIVTVSLLEVFSFCS.

Bcl-2 :

MAHAGRTGYDNREIVMKYIHYKLSQRGYEWDAGDVGAAPPGAAPAFTPGIFSS
QPGHTPHPAASRDPVARTSPLQTPAAPGAAAGPALSPVPPVVHLALRQAGDDF
FTSRRYRGDFAEMSSQLHLTPFTARGRFATVVEELFRDGVNWGRIVAFFEFGGV
MCVESVNFTREMSPLVDNIALWMTEYLNRHLHTWIQDNGGWVGASGDVSLG.

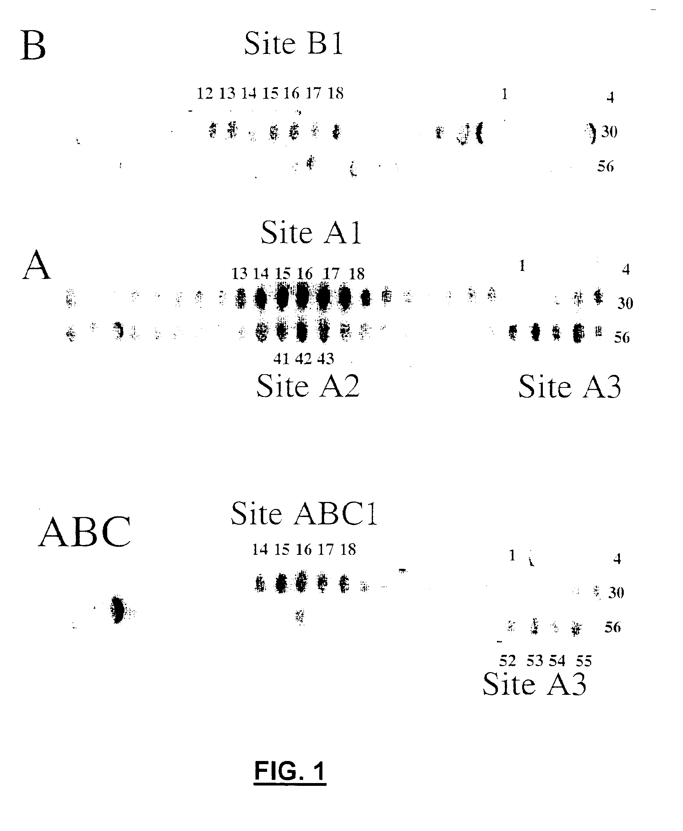

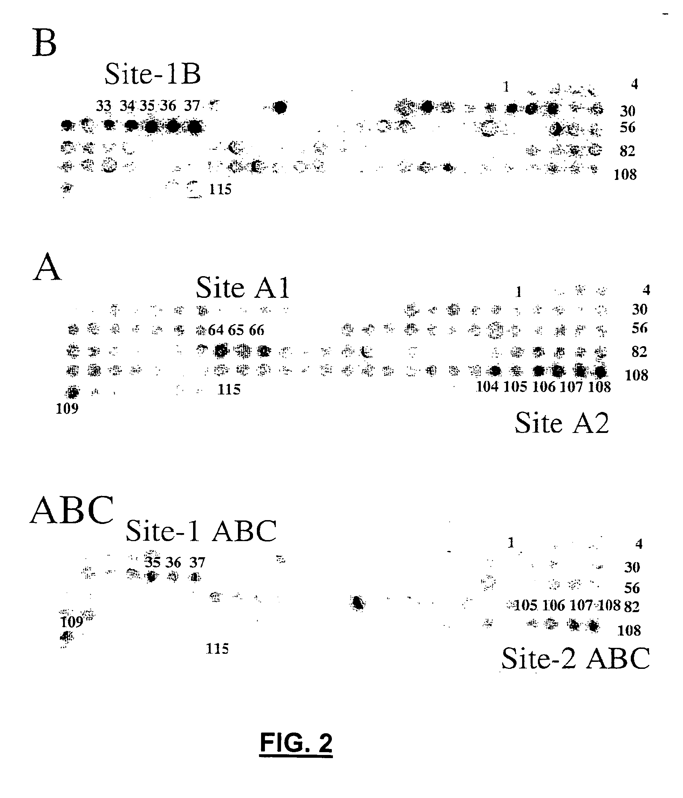

L'interaction spécifique de chaque protéine purifiée (respectivement la sous-unité structurale A, la sous-unité régulatrice B55(B) ou l'holoenzyme trimérique ABC (appelé PP2A1) avec une séquence peptidique est révélée, comme un western blot, après incubation de la membrane avec un anti-corps dirigé contre la protéine structurale A (A), la sous-unité régulatrice B (B) et avec un mélange d'anticorps reconnaissant les protéines A,B, et C de PP2A (ABC) (Figures 1,2).

### EXEMPLE 1

### Identification de séquences peptidiques contenant des sites de liaison de la protéine E4orf4 codée par l'adénovirus de type 2 de chien avec des protéines de la famille des PP2A.

Le criblage d'une membrane contenant les peptides recouvrant les séquences de E4orf4 avec les différentes formes de PP2A purifiées (figure 1) nous a permis d'identifier cinq séquences d'acides aminés qui contiennent des sites d'interaction de E4orf4 avec PP2A (cf. tableau 1).

Nous avons respectivement pu déterminer:
- une séquence peptidique contenant un site de liaison de E4orf4 avec la sous-unité structurale B ("site B1" correspondant aux peptides 12 à 18).
- Trois séquences peptidiques correspondant à trois sites de liaison de E4orf4 avec la sous-unité A ("site A 1" correspondant aux peptides 13 à18 et "site A2" correspondant aux peptides 41 à 43 et "site A3" correspondant aux peptides 52 à 55).
- Deux séquences peptidiques correspondant à deux sites de liaison de E4orf4 avec la protéine PP2A1 ("site B1" correspondant aux peptides 14 à18 et "site A3" correspondant aux peptides 52 à 55).

Il est intéressant de constater (tableau 1) que les sites B1, A1 et ABC1 se recouvrent partiellement ce qui suggère:
- que les sous-unités A et B peuvent interagir au même site ce qui n'a jamais été établi dans le système PP2A. Par ailleurs, l'interaction de PP2A trimérique à ce site requiert une séquence un peu plus courte ce qui suggère une régulation conformationnelle.

**Tableau 1: Sites de liaisons de E4orf4 avec différentes PP2A**

| | |
|---|---|
| **Site B1** | **23**- RELGSMLE SPMEFLFDTI DDVTAS-**46** |
| **Site A1** | **25**- LGSMLE SPMEFLFDTI DDVTAS-**46** |
| **Site ABC1** | **28** -SMLE SPMEFLFDTI DDVTAS-**4** |
| | |
| **Site A2** | **83**- HFKGTLIKEISDVVNN-**98** |
| | |
| **Site A3** | **106**-AFRGREVTVSLLEVFSFC-**124** |

### EXEMPLE 2

### Identification de séquences peptidiques contenant des sites de liaison de la protéine Bcl-2 avec des protéines de la famille des PP2A.

Le criblage d'une membrane contenant les peptides recouvrant les séquences de Bcl-2 avec les différentes formes de PP2A purifiées (figure 2) nous a permis d'identifier cinq séquences d'acides aminés qui contiennent des sites d'interaction de Bcl-2 avec PP2A (cf. tableau 2).

Nous avons respectivement pu déterminer:
- une séquence peptidique contenant un site de liaison de Bcl-2 avec la sous-unité régulatrice B (site 1-B correspondant aux peptides 33-37).
- deux séquences peptidiques contenant deux sites de liaison de Bcl-2 avec la sous-unité structurale A (site A1 correspondant aux peptides 64-67 et site A2 correspondant aux peptides 104-109 ).
- deux séquences peptidiques contenant un site de liaison de Bcl-2 avec avec l'holoenzyme PP2A1 (site 1-ABC correspondant aux peptides 35 à 37 et site 2-ABC correspondant aux peptides 105-109).

Il est intéressant de constater que les sites 1-ABC et 2-ABC correspondent respectivement aux sites 1-B et A2 avec respectivement des décalages de deux et quatre acides aminés probablement liés à une conformation différente résultant de l'interaction des protéines A ou B dans l'holoenzyme.

Par ailleurs, il est notable que le site 1-B/1-ABC se situe au niveau de la ser-70 dont la phosphorylation régule l'activité de PP2A alors que le site 2-ABC/A2 se situe à l'extrémité C-terminale de la protéine. Au total, et contrairement aux sites d'interaction avec la PP1 ces deux sites n'interfèrent pas avec les domaines BH de la protéine PP2A (voir Garcia A. et al. PP1 et PP2A des ser/thr phosphatases au coeur de l'apoptose (2001). Med/Sci 17, 1214-1216 pour une discussion générale).

**Tableau 2: Sites de liaisons de Bcl-2 avec différentes PP2A**

| | |
|---|---|
| **Site 1-B** | **67**-ARTSPLQTPAAPGAAAGPAL-**86** |
| **Site A1** | **129**- RFATVVEELFRDGVNW-**144** |
| **Site A2** | **207**- RPLFDFSWLSLKTLLSLALVGA-**228** |
| | |
| **Site 1-ABC** (site1 B) | 71- PLQTPAAPGAAAGPAL -**86** |
| | |
| **Site 2-ABC** (site A2) | **209**- LFDFSWLSLKTLLSLALVGA-**228** |

## Revendications

1. Peptide d'une taille inférieure à 30 acides aminés, de préférence inférieure à 20 acides aminés, **caractérisé en ce qu'**il lie *in vitro,* de manière spécifique, une holoenzyme protéine phosphatase de type 2A ou l'une de ses sous-unités, et **en ce qu'**il est inclus :
- dans l'une des séquences suivantes issues de la protéine E4orf4 de l'adénovirus de type 2 de chien:
a) HFKGTLIKEISDVVNN (SEQ. ID NO:4)
b) AFRGREVTVSLLEVFSFC (SEQ. ID NO: 5) ; ou
- dans une séquence se distinguant de SEQ ID NO: 4 ou SEQ ID NO: 5 par substitution ou délétion d'acides aminés, ladite séquence distincte conservant néanmoins les propriétés de liaison pour la protéine phosphatase de type 2A ou l'une de ses sous-unités.

2. Peptide selon la revendication 1, **caractérisé en ce que** son administration induit l'apoptose des cellules et en particulier de cellules tumorales.

3. Peptide selon la revendication 1 ou 2, **caractérisé en ce qu'**il inhibe, de manière compétitive, l'interaction de la protéine native dont il est issu avec une holoenzyme PP2A ou l'une de ses sous-unités.

4. Peptide selon l'une des revendications 1 à 3, **caractérisé en ce qu'**il est couplé à un vecteur capable de transférer ledit peptide dans une cellule eucaryote.

5. Polypeptide **caractérisé en ce qu'**il est constitué de la répétition d'un peptide selon l'une quelconque des revendications 1 à 4.

6. Polynucléotide **caractérisé en ce que** sa séquence consiste en la séquence codante d'un peptide selon l'une quelconque des revendications 1 à 4.

7. Polynucléotide **caractérisé en ce qu'**il consiste en un multimère du polynucléotide selon la revendication 6.

8. Vecteur d'expression cellulaire, **caractérisé en ce qu'**il comporte un polynucléotide selon la revendication 6 ou 7, et des séquences régulatrices permettant l'expression d'un peptide selon l'une quelconque des revendications 1 à 4 dans une cellule hôte.

9. Anticorps purifié polyclonal ou monoclonal **caractérisé en ce qu'**il est capable de lier de façon spécifique l'un quelconque des peptides selon l'une des revendications 1 à 4.

10. Composition pharmaceutique comprenant l'un des peptides selon l'une des revendications 1 à 4, en combinaison avec un véhicule pharmaceutiquement acceptable.

11. Composition pharmaceutique comprenant un des éléments choisis parmi un polynucléotide selon la revendication 6 ou 7, un vecteur d'expression selon la revendication 8 ou un anticorps selon la revendication 9.

12. Utilisation des peptides définis selon l'une des revendications 1 à 4, dans la préparation d'un médicament utile dans le traitement de tumeurs.

13. Utilisation d'un peptide défini selon l'une des revendications 1 à 4, dans la préparation d'un médicament apte à induire l'apoptose de cellules cibles, et en particulier de cellules tumorales.

14. Utilisation d'un peptide défini selon l'une des revendications 1 à 4, dans la préparation d'un médicament utile dans le traitement du cancer.

15. Utilisation d'un polynucléotide selon l'une des revendications 1 à 4 ou d'un anticorps selon la revendication 9 dans le diagnostic *in vitro* du cancer.
